# FASCICULE DE BREVET EUROPEEN

(11) **EP 1 402 269 B1**
(45) Date de publication et mention de la délivrance du brevet: **14.03.2007**
(21) Numéro de dépôt: 02755095.3
(22) Date de dépôt: 02.07.2002
(51) Int. Cl.: G01N 33/68, C12Q 1/37, G01N 33/566

(54) **PROCEDE DE SEPARATION ET/OU DETECTION ET/OU IDENTIFICATION ET/OU QUANTIFICATION DE PROTEINES PRIONS**
VERFAHREN ZUR TRENNUNG UND/ODER ZUM NACHWEIS UND/ODER ZUR IDENTIFIZIERUNG UND/ODER ZUR QUANTIFIZIERUNG VON PRIONPROTEINEN
METHOD FOR SEPARATING AND/OR DETECTING AND/OR IDENTIFYING AND/OR QUANTIFYING PRION PROTEINS

(30) Priorité: 03.07.2001 FR 0108797
(43) Date de publication de la demande: 31.03.2004
(73) Titulaire: Apoh Technologies SA, 34000 Montpellier (FR)
(72) Inventeur: STEFAS, Elie, F-34280 La Grande-Motte (FR)
(74) Mandataire: Laget, Jean-Loup
(86) Numéro de dépôt international: PCT/FR2002/002292
(87) Numéro de publication internationale: WO 2003/005037

(56) Documents cités:
- WO-A-94/18569
- WO-A-96/17249
- FR-A- 2 690 444
- FR-A- 2 701 263
- HOCHSTRASSER D F ET AL: "Elevation of apolipoprotein E in the CSF of cattle affected by BSE" FEBS LETTERS, ELSEVIER SCIENCE PUBLISHERS, AMSTERDAM, NL, vol. 416, no. 2, 20 octobre 1997 (1997-10-20), pages 161-163, XP004261330 ISSN: 0014-5793
- DANDOY-DRON R ET AL: "GENE EXPRESSION IN SCRAPIE CLONING OF A NEW SCRAPIE-RESPONSIVE GENE AND THE IDENTIFICATION OF INCREASED LEVELS OF SEVEN OTHER MRNA TRANSCRIPTS" JOURNAL OF BIOLOGICAL CHEMISTRY, AMERICAN SOCIETY OF BIOLOGICAL CHEMISTS, BALTIMORE, MD, US, vol. 273, no. 13, 27 mars 1998 (1998-03-27), pages 7691-7697, XP000941399 ISSN: 0021-9258

## Description

La présente invention concerne un procédé de séparation et/ou de détection et/ou d'identification et/ou de quantification de protéines prions, responsables de maladies neurodégénératives, dans divers matériaux biologiques.

Les prions font partie des "agents transmissibles non conventionnels" (ATNC) et sont impliqués dans des maladies rencontrées chez l'homme et les animaux. Il s'agit d'agents responsables de maladies neurodégénératives regroupées sous le nom d'encéphalopathies subaiguës spongiformes transmissibles (ESST). Ces encéphalopathies, reliées aux prions, incluent la tremblante du mouton et de la chèvre, l'encéphalopathie bovine ou maladie de la vache folle, la maladie du dépérissement chronique des ruminants sauvages, les encéphalopathies des visons et des chats, chez l'animal, la maladie de Creutzfeld-Jacob (MJC), le syndrome de Gerstmann-Straüssler-Scheinker (GSS), le kuru et l'insomnie familiale fatale, chez l'homme (Les virus transmissibles de la mère à l'enfant, Ed. John Libbey Eurotext, 1999, Ermias D. Belay, Annu. Rev. Microbiol., 1999, 53: 283-314).

Ces maladies, toujours mortelles, posent un grave problème de santé publique notamment du fait des difficultés que l'on rencontre pour l'identification et la détection précoce de ces agents. Il y a donc une forte demande relative à des procédés fiables de diagnostic et de mesures thérapeutiques efficaces, de ces agents transmissibles non conventionnels.

Ces maladies semblent être causées par la transition post-traductionnelle et conformationnelle d'une protéine prion cellulaire, normale, ci-après désignée PrP^{C}, en une forme pathogénique, anormale, ci-après désignée PrP^{SC} (Cohen F.E. and Prusiner S.B., Annu. Rev. Biochem., 1998, 67: 793-819).

La forme cellulaire normale de la protéine prion est une glycoprotéine de surface cellulaire, hautement conservée et exprimée par un large spectre de cellules, en particulier par des cellules neuronales. Sa présence est indispensable pour que la maladie puisse se produire. Dans les encéphalopathies spongiformes transmissibles, cette molécule est convertie en une forme modifiée, du point de vue conformationnel, présentant une résistance partielle à la protéolyse. Ainsi, dans le cerveau des animaux ou des humains présentant des ESST, on observe une accumulation de PrP^{SC} anormale sous forme de fibrilles et dans certains cas sous forme de dépôts amyloïdes dans les cellules. Cette PrP^{SC} anormale a un poids moléculaire compris entre 33 et 35 kDa avant protéolyse et a un poids moléculaire compris entre 27 et 30 kDa après action de la protéinase K : cette résistance à la protéinase K permet de différencier la PrP^{SC} de la PrP^{C} qui est détruite par l'action de ladite protéinase. Des études biophysiques ont également démontré que la PrP^{C} contient un nombre élevé d'hélices α (42%) et très peu de feuillets β, alors qu'au contraire la forme PrP^{SC} contient moins d'hélices α (30%) et un nombre élevé de feuillets β (43%) et a une tendance à se polymériser sous forme de fibrilles amyloïdes (Cohen F.E. and Prusiner S.B., Annu. Rev. Biochem., 1998, 67: 793-819). La protéine prion, désignée d'une manière générale par PrP, est également caractérisée par son affinité pour les polyanions polysulfatés tels que l'héparine sulfate et le dermatan sulfate (Brimacombe B. et al., Biochem. J., 1999, 342: 605-613).

On sait que la β2-glycoprotéine I, ci-après en abrégé β2GPI, est une glycoprotéine plasmatique, dont la séquence a été notamment indiquée dans les articles de J. LOZIER et coll., Proc. Natl. Acad. Sci. USA, Vol. 81, pages 3640-3644, Juillet 1984 et de T. KRISTENSEN et coll., FEBS Letters, Vol. 289, 1991, pages 183-186. La β2GPI est également appelée Apolipoprotéine H (APOH). Il a été constaté que cette protéine présente un polymorphisme structural : la dénomination β2GPI sera ci-après considérée comme générique pour toutes les formes.

Ce polymorphisme structural est sous contrôle génétique comme ceci a été notamment indiqué dans l'article de DK. SANGHERA et coll., Hum. Genet., Vol. 100, 1997, pages 57-62. Il est dû à la présence de quatre allèles: trois allèles fréquents (APOH*1, APOH*2 et APOH*3) et un allèle rare (APOH*4). L'allèle APOH*3 a été par la suite sous-typé en APOH*3^{W} et APOH*3^{D} en se basant sur sa réactivité avec un anticorps monoclonal 3D11. Ce polymorphisme est dû à plusieurs substitutions dans la région ADN codant pour l'APOH telles que Ser88Asn (DK. SANGHERA et coll., Hum. Genet., Vol. 100, 1997, pages 57-62), Val247Leu (A. STEINKASSERER et coll., Hum. Genet., Vol. 91, 1993, pages 401-402), Cys306Gly (DK. SANGHERA et coll., Hum. Mol. Genet., Vol. 6, 1997, pages 311-316), et Trp316Ser (DK. SANGHERA et coll., Hum. Genet., Vol. 100, 1997, pages 57-62 et DK. SANGHERA et coll., Hum. Mol. Genet., Vol. 6, 1997, pages 311-316). Les mutations Ser88Asn et Trp316Ser correspondent aux allèles APOH*1 et APOH*3^{W} respectivement. La forme β2'GPI décrite dans FR-2 701 260 B 1 résulte de la mutation Thr318Ser.

La β2GPI est connue comme une glycoprotéine ayant une forte affinité pour les phospholipides anioniques tels que la cardiolipine (H. WURM, Int. J. Biochem., Vol. 16, 1984, pages 511-515).

Dans la demande internationale WO 94/18569, on a indiqué que des composés viraux se fixaient de façon spécifique sur une forme de β2GPI, à savoir celle décrite dans la demande de brevet français 2 701 263, que cette forme de β2GPI soit à l'état pur ou dans une composition protéinique la contenant ; cette forme de β2GPI est isolée à partir du résidu fixé sur la (les) colonne(s) de chromatographie d'affinité utilisée(s) dans le procédé de purification de l'albumine du plasma sanguin décrit dans FR-A-2 690 444 ; elle a un poids moléculaire de 50 000 ± 3 000 daltons.

On a formulé l'hypothèse que la liaison de la β2GPI à des composés viraux pourrait impliquer les phospholipides présents sur ces composés (H. MEHDI et coll., J. Virol., Vol. 68 (4), 1994, pages 2415-2424, AR.NEURATH et coll., Virology, Vol. 204 (1), 1994, pages 475-477, E. STEFAS et coll., AIDS Res. Hum. Retr., Vol. 13 (1), 1997, pages 97-104, E. STEFAS et coll., Hepatology, Vol. 33 (1), 2001, pages 207-217). Il a également été décrit que la β2GPI sert de cofacteur pour la liaison des anticorps anti-phospholipides aux phospholipides anioniques (M. GALLI et coll., Lancet, Vol. 335 (8705), 1990, pages 1544-1547, HP. McNEIL et coll., Proc. Natl. Acad. Sci. USA, Vol. 87 (11), 1990, pages 4120-4124). On a aussi émis l'hypothèse, dans la littérature, que les résidus lysine de la β2GPI étaient responsables de la liaison de cette dernière aux phospholipides anioniques (Steinkasserer A. et coll., Biochemical Journal, 1991, 277 (Pt 2) : 387-391) ; cette hypothèse était confortée par le fait que la modification desdits résidus lysine par carbamylation abolissait sa liaison aux phospholipides (Arvieux J. et coll., Thrombosis and Haemostasis, 70 (2), 1993: 336-341, Kertesz Z. et coll., Biochemical Journal, 310, 1995 : 315-321).

Il semble, en conséquence, que la liaison entre un composé infectieux et la β2GPI fait intervenir des interactions protéine-phospholipide, ainsi que d'autres types de liaison, notamment basés sur des interactions protéine-protéine.

Il apparaît clairement, à partir des données de la littérature médicale, qu'il y a un besoin urgent de développer des méthodes d'isolement, d'identification et de diagnostic d'agents pathogènes tels que des protéines prions anormales PrP^{SC}, causant des maladies connues ainsi que des maladies émergentes, et des méthodes d'isolement, d'identification de protéines prions normales PrP^{C} dont les PrP^{SC} dérivent Ces méthodes permettront à terme de mieux connaître ces agents pathogènes ainsi que les mécanismes qu'ils mettent en jeu pour infecter des organismes vivants.

Par conséquent, le but de la présente invention est de fournir :
- un procédé de séparation de protéine prion d'un matériau biologique, et/ou
- un procédé d'isolement et purification de protéine prion, et/ou
- un procédé de détection de protéine prion dans un matériau biologique, et/ou
- un procédé d'identification de protéine prion dans un matériau biologique, et/ou
- un procédé de quantification de protéine prion dans un matériau biologique.

Selon l'invention, on a constaté que, de façon surprenante et inattendue, des protéines prions PrP peuvent être séparées et/ou détectées et/ou identifiées et/ou quantifiées par l'intermédiaire de leur liaison aux différentes formes de β2GPI. Le terme de "liaison" indique que ces PrP sont physiquement connectées à, et interagissent avec, les différentes formes de β2GPI. Cette liaison peut être démontrée par n'importe quel procédé ou essai connu en la matière tels que les essais utilisant la biotine et l'avidine ou la streptavidine, de type immunoenzymatique, de type ELISA ou Immunoblot, de type radioimmunologique, de type RIA, des essais de compétition, des essais d'agglutination, des essais d'immunoprécipitation, des essais de chromatographie... De façon générale, on appellera ici "complexe" une association directe ou indirecte, entre au moins une PrP, normale ou anormale, et au moins une forme de β2GPI ; ces complexes seront, de façon générale, désignés ci-après par la notation "PrP/β2GPI".

Dans la présente demande de brevet, on entend, de façon générique, par PrP aussi bien les composés protéiniques, constitutifs d'une PrP, que des particules de type PrP. Les particules de type PrP sont, soit des PrP complètes ou incomplètes, soit des parties de PrP, soit des assemblages contenant des composés constitutifs de PrP, qui présentent certaines propriétés des PrP ou des composés PrP, en particulier, celles d'être détectées par certains anticorps spécifiques de composés PrP.

Selon la présente invention, on entend par "matériau biologique", un tissu biologique, une préparation ou un extrait issu du tissu biologique, liquide ou solide, ou un milieu naturel, liquide ou solide, susceptible de contenir ou porter une PrP au sens ci-dessus défini. Le matériau peut ainsi être un mélange d'au moins deux matériaux tels que ci-dessus définis. Un tel matériau biologique peut donc être, notamment, soit préparé à partir de tissus, d'organes, de selles ou de liquides biologiques d'un malade atteint d'une infection due à une PrP^{SC}, soit obtenu à partir de cultures "in vitro"; un tel matériau biologique peut aussi être un sérum, du plasma, de l'urine, du liquide céphalo-rachidien, du liquide synovial, du liquide péritonéal, du liquide pleural, du liquide séminal, de la salive, des sécrétions gastriques, du mucus, du liquide ascitique ou autres.

La présente invention a donc pour objet un procédé de séparation et/ou de détection et/ou d'identification et/ou de quantification dans un matériau biologique d'au moins une protéine prion (PrP), caractérisé par le fait qu'il comprend une étape de séparation et/ou de détection et/ou d'identification et/ou de quantification d'un complexe (PrP/β2GPI) formé d'au moins une protéine prion liée à au moins une forme de β2-glycoprotéine I (β2GPI).

Selon une particularité, le procédé comprend une étape de séparation et/ou de détection et/ou d'identification et/ou de quantification d'un complexe (PrP^{SC}/β2GPI) formé d'au moins une protéine prion anormale (PrP^{SC}) liée à au moins une forme de β2GPI, ledit procédé constituant un procédé de séparation et/ou de détection et/ou d'identification et/ou de quantification dans un matériau biologique d'au moins une protéine prion anormale. En particulier on peut utiliser pour former un complexe (PrP^{SC}/β2GPI) au moins une protéine prion anormale PrP^{SC} provenant de la tremblante du mouton ou de la chèvre, de l'encéphalopathie bovine, de la maladie du dépérissement chronique des ruminants sauvages, des encéphalopathies des visons ou des chats, de la maladie de Creutzfeld-Jacob (MJC), du syndrome de Gerstmann-Straüssler-Scheinker (GSS), du kuru ou de l'insomnie familiale fatale. En outre, on peut utiliser, pour former un complexe (PrP/β2GPI), au moins une β2GPI d'origine humaine, ou d'origine animale, une β2GPI recombinante, ou une β2GPI obtenue par synthèse chimique ou une forme modifiée de β2GPI.

Avantageusement, avant l'étape de séparation et/ou de détection et/ou d'identification et/ou de quantification du complexe (PrP^{SC}/β2GPI), on soumet le matériau biologique à l'action de détergents et/ou d'enzymes, en particulier à l'action de la protéinase K.

Selon une première mise en oeuvre de l'invention, on réalise une étape de fixation de PrP contenue dans un matériau biologique à au moins une forme de β2GPI intentionnellement rajoutée audit matériau biologique pour former ledit complexe, suivie d'une étape de séparation et/ou de détection et/ou d'identification et/ou de quantification du complexe (PrP/β2GPI).

Selon un mode de réalisation, on réalise une étape de fixation sur un support, d'au moins une forme de β2GPI ou de ladite (ou desdites) PrP, avant ou après l'étape de fixation de ladite (ou desdites) PrP à ladite (ou auxdites) forme(s) de β2GPI pour former ledit complexe, une étape de séparation consistant à séparer le matériau biologique du support sur lequel est fixé le complexe, une étape de détection et/ou d'identification et/ou de quantification consistant, après ladite étape de séparation, à détecter et/ou à identifier et/ou à quantifier le complexe fixé au support par sa partie qui n'est pas liée au support. Avantageusement, on utilise, comme support, un support solide.

Selon une variante de réalisation, la fixation sur le support est réalisée par l'intermédiaire d'un composé se liant à l'une des parties PrP ou β2GPI du complexe, ladite étape de détection et/ou d'identification et/ou de quantification consistant à détecter et/ou à identifier et/ou à quantifier le complexe par sa partie qui n'est pas liée au support. Avantageusement, le composé se liant à la β2GPI ou à la PrP est un anticorps reconnaissant respectivement la β2GPI ou la PrP, ou bien une autre protéine, un composé biologique, un composé chimique ou un détergent se fixant à la PrP ou à la β2GPI.

On peut réaliser l'étape de fixation d'au moins une forme de β2-GPI ou de la (ou des) PrP sur un support, par réaction de groupes réactifs de la (ou des) forme(s) de β2GPI ou de la (ou des) PrP avec des sites réactifs du support, ladite (ou lesdites) forme(s) étant mise(s) en solution dans un tampon ayant un pH compris entre 2,5 et 10,5, de préférence entre 5,5 et 7,5, pour obtenir une solution ayant une concentration comprise entre 0,01 et 100g/l de forme(s) de β2GPI ou de PrP, le support étant maintenu en contact avec la solution à une température comprise entre 0° et 40°C pendant un temps d'incubation compris entre 10 secondes et 24 heures, puis la séparation du support et de la solution par lavage du support.

L'étape de fixation d'au moins une PrP à au moins une forme de β2GPI pour former un complexe peut être réalisée par mise en contact d'au moins une forme de β2GPI, avec le matériau biologique susceptible de contenir des PrP à une température comprise entre 0° et 50°C, avantageusement voisine de 37°C, pendant une période de temps comprise entre 10 secondes et 24 heures, le matériau biologique étant dilué à l'aide d'un tampon donnant un pH compris entre 3,5 et 10, de préférence compris entre 5,6 et 7,6.

On peut effectuer la détection et/ou l'identification et/ou la quantification de la (ou des) PrP du complexe par des anticorps spécifiques de la (ou des) PrP, ou par des procédés d'infection de cellules ou d'organismes susceptibles à l'infection des PrP ; avantageusement, on effectue la détection et/ou l'identification et/ou la quantification de la (ou des) PrP du complexe par un anticorps reconnaissant spécifiquement un antigène, de préférence de nature protéique, de la (ou des) PrP.

On peut aussi, selon une autre façon d'opérer, effectuer la détection et/ou l'identification et/ou la quantification de la β2GPI du complexe par des anticorps spécifiques de la β2GPI.

Avantageusement, on couple l'anticorps utilisé à un marqueur enzymatique, de l'or colloïdal, à un traceur radioactif, fluorescent ou luminescent. On peut coupler l'anticorps à un marqueur enzymatique, dont l'enzyme est mise en contact avec un substrat spécifique apte à se transformer en un produit coloré.

Quand on fixe le complexe au support par l'intermédiaire de sa partie β2GPI, l'étape de séparation comprend l'isolement de la PrP du complexe fixé au support par un procédé d'élution de chromatographie d'affinité. Avantageusement, ledit isolement est réalisé par élution de la PrP fixée au support solide à l'aide d'un tampon ayant un pH compris entre 2 et 10,5, une concentration en NaCl comprise entre 0 et 5M, de préférence à l'aide d'un tampon glycine-HCl 0,1 mole/litre ayant un pH de 2,5.

Selon une particularité de l'invention, la séparation du support et de la solution est suivie d'une étape de saturation des sites actifs du support, en faisant réagir sur les sites actifs une solution d'albumine sérique bovine ou de caséine.

Selon une autre mise en oeuvre de l'invention, le procédé de séparation et/ou de détection et/ou d'identification et/ou de quantification d'au moins une PrP dans un matériau biologique qui contient naturellement au moins une forme de β2GPI, comprend une étape de séparation et/ou de détection et/ou d'identification et/ou de quantification d'un complexe (PrP/β2GPI) formé d'au moins une PrP liée à au moins une forme de β2GPI naturellement présente dans ledit matériau.

Les formes de β2GPI utilisées selon la présente invention, pour former les complexes (PrP/β2GPI), peuvent, comme ci-dessus indiqué, être d'origine humaine, animale, recombinante, ou obtenues par voie chimique ou des formes modifiées de β2GPI.

Le terme "d'origine humaine" se réfère à n'importe quelle forme naturelle de β2GPI trouvée chez l'homme ou obtenue après culture de cellules humaines, ou à des fragments (combinaison de quelques acides aminés, tels que des polypeptides ou peptides) de celle-ci, obtenus soit en cours de purification, par coupure(s) enzymatique(s) provoquée(s) par des enzymes déjà présentes dans les liquides biologiques, soit après purification à l'aide d'enzymes spécifiques ou non de sites présents sur ces formes naturelles humaines.

Le terme "d'origine animale" se réfère à n'importe quelle forme naturelle de β2GPI trouvée chez l'animal, ou obtenue après culture de cellules animales, ou à des fragments (combinaison de quelques acides aminés, tels que des polypeptides ou peptides) de celle-ci, obtenus soit en cours de purification, par coupure(s) enzymatique(s) provoquée(s) par des enzymes déjà présentes dans les liquides biologiques, soit après purification à l'aide d'enzymes spécifiques ou non de sites présents sur ces formes naturelles animales.

Le terme "d'origine recombinante" se réfère ou bien à n'importe quelle forme recombinante de β2GPI obtenue selon les techniques de recombinaison d'ADN telles que décrites par Maniatis (MANLATIS T. et coll., Molecular cloning: a laboratory manual. Cold Spring Harbor Laboratory Press, Cold Spring Harbor, NY, 1982), après insertion du gène, modifié ou non, et recombinaison génétique, ou après modification du gène déjà exprimé par des procédés connus en la matière chez les bactéries ou autres cellules utilisées dans la production de protéines recombinantes, ou bien à des fragments (combinaison de quelques acides aminés, tels que des polypeptides ou peptides) de telles formes recombinantes, obtenus soit en cours de purification, par coupure(s) enzymatique(s) provoquée(s) par des enzymes déjà présentes dans les liquides biologiques, soit après purification à l'aide d'enzymes spécifiques ou non de sites présents sur ces formes recombinantes.

Le terme "modifié" se réfère à n'importe quelle forme naturelle de β2GPI trouvée chez l'homme ou l'animal, à n'importe quelle forme d'origine recombinante, à n'importe quelle forme obtenue après culture de cellules humaines ou animales, ou encore à des fragments (combinaison de quelques acides aminés, tels que des polypeptides ou peptides) de ces formes, obtenus soit en cours de purification, par coupure(s) enzymatique(s) provoquée(s) par des enzymes déjà présentes dans les liquides biologiques, soit après purification à l'aide d'enzymes spécifiques ou non de sites présents sur lesdites formes, après que toutes les formes susmentionnées aient subi des modifications, par exemple par voie chimique, de certains de leurs acides aminés, comme par exemple la carbamylation des résidus lysine.

Le terme "obtenue par voie chimique" se réfère à n'importe quelle forme de β2GPI d'origine humaine, animale ou recombinante telle que définie ci-dessus obtenue par une synthèse chimique. En particulier, les polypeptides et peptides provenant desdites formes de β2GPI peuvent être préparés selon n'importe quel procédé connu en la matière notamment par synthèse chimique classique comme décrit par Atherton et Shepard dans "Solid phase peptide synthesis", IRL Press, Oxford, 1989. Il est clair que les termes "polypeptides" et "peptides" se réfèrent à un polymère d'acides aminés comprenant moins d'acides aminés que la séquence de la protéine naturelle, mais n'exclut pas les modifications post-traductionnelles des polypeptides et peptides, telles que la glycosylation, l'acylation, la phosphorylation, les modifications avec des acides gras ou autres. Sont également inclus dans la définition, des polypeptides et des peptides avec des substitutions au niveau des acides aminés, des versions mutées ou des variations de la séquence naturelle de ces polypeptides et peptides, des polypeptides et peptides avec des liaisons substituées, des polypeptides et peptides contenant des résidus cystéine reliés par des ponts disulfure, des résidus cystéine sans ponts disulfure, aussi bien que d'autres modifications connues en la matière.

Selon la présente invention, on peut utiliser, comme forme de β2GPI, la β2GPI pure ou sous forme de composition protéinique contenant, en particulier, d'autres glycoprotéines. Elle peut ainsi être obtenue :
- à partir du plasma ou autre liquide biologique, par exemple sérum, urine, liquide céphalo-rachidien, humain ou animal, en mettant en oeuvre des procédés de purification déjà décrits dans la littérature ou dans le brevet français 2 701 263 ; ou
- à partir du commerce ; ou
- à partir de surnageants de cellules immortalisées qui l'expriment ; ou
- par expression du gène qui la code dans des bactéries ou autres cellules utilisées dans la production de protéines recombinantes ; ou
- par synthèse chimique.

Les formes de β2GPI peuvent être caractérisées et séquencées selon n'importe quel procédé connu en la matière.

Pour mettre en oeuvre le procédé selon l'invention, on peut ou bien effectuer la détection et/ou l'identification et/ou la quantification de PrP sans fixation préalable du complexe (PrP/β2GPI) sur un support ou bien effectuer la séparation et/ou détection et/ou identification et/ou quantification de PrP avec fixation dudit complexe sur un support par un élément constitutif du complexe ; dans le premier cas, la détection et/ou l'identification et/ou la quantification s'effectue dans le milieu où le complexe s'est formé, soit après fixation dudit milieu par une méthode physique, chimique ou biochimique, par exemple sur une surface, soit sans fixation dudit milieu ; dans le deuxième cas, le support peut, avantageusement, être un support solide, la séparation consistant à séparer le support sur lequel est fixé le complexe, la détection et/ou l'identification et/ou la quantification consistant à détecter et/ou identifier et/ou quantifier le complexe fixé au support après avoir séparé ledit support du matériau biologique.

Le terme "support solide" se réfère à n'importe quel support solide connu en la matière tel qu'un de ceux décrits dans "Current Protocols in Immunology" aux Editions Coligan J., Kruisbeek A., Margulies D., Shevach E et Strober W., Wiley Interscience, 1992. Ce support peut, par exemple, être une plaque de microtitration type ELISA, une membrane, notamment de nitrocellulose, un gel de chromatographie, des billes, notamment en polystyrène, des tubes, notamment en polystyrène ou polypropylène, ou des cellules vivantes, humaines ou animales ou bactériennes ou virales.

Selon un premier mode de mise en oeuvre de l'invention dans le cas où l'on fixe le complexe sur un support, on retient le complexe (PrP/β2GPI) sur le support par l'intermédiaire de la partie β2GPI du complexe ; ensuite, la partie du complexe correspondant à la PrP est détectée/identifiée/quantifiée ou isolée par tout moyen approprié. La fixation de la partie β2GPI sur le support peut être effectuée après la formation du complexe ou, de préférence, avant la formation du complexe. Si la fixation de la partie β2GPI est effectuée avant la formation du complexe, ladite fixation sur le support solide se fait par réaction de groupes réactifs de la (ou des) forme(s) de la β2GPI avec des sites réactifs du support selon n'importe quel procédé connu en la matière, tel que décrit dans "Current Protocols in Immunology" aux Editions Coligan J., Kruisbeek A., Margulies D., Shevach E et Strober W., Wiley Interscience, 1992. Cette réaction est, de préférence, effectuée à une température comprise entre 0° et 40°C, la (ou les) forme(s) de β2GPI étant, de préférence, mise(s) dans un tampon ayant un pH compris entre 2,5 et 10,5, avantageusement entre 5,5 et 7,5. On utilise, de préférence, un tampon isotonique ou presque isotonique. Le tampon peut être du type phosphate ou acétate. La solution obtenue a avantageusement une concentration comprise entre 0,01 et 100 g/l de forme(s) de β2GPI. Le support est avantageusement maintenu en contact avec le tampon contenant la (ou les) forme(s) de β2GPI à une température comprise entre 0 et 40°C et pendant un temps d'incubation compris entre 10 secondes et 24 heures. Après incubation, on sépare du support le tampon contenant la (ou les) forme(s) de β2GPI n'ayant pas réagi et on effectue un lavage du support, de préférence, avec le même tampon que celui qui contenait la (ou les) forme(s) de β2GPI. Il peut être nécessaire de saturer les sites actifs du support, qui n'ont pas réagi, avec la (ou les) forme(s) de β2GPI. Dans ce cas, on fait réagir sur ces sites actifs d'autres groupes actifs choisis parmi les solutions d'albumine bovine, de sérum de veau foetal, de caséine ou similaires. On utilise avantageusement, dans ce but, une solution d'albumine sérique bovine, en particulier une solution à 2% dans le tampon utilisé pour la (ou les) forme(s) de β2GPI. Après réaction, le support est, de préférence, rincé et séché.

La réaction du support solide portant une (ou des) forme(s) de β2GPI avec le matériau biologique se fait selon n'importe quel procédé connu en la matière tels que ceux décrits dans "Current Protocols in Immunology" aux Editions Coligan J., Kruisbeek A., Margulies D., Shevach E et Strober W., Wiley Interscience, 1992. Le support sur lequel est (sont) fixée(s) la (ou les) forme(s) de β2GPI est ensuite mis en contact avec un matériau biologique susceptible de contenir des PrP. On dilue, de préférence, le matériau biologique à l'aide d'un tampon donnant un pH compris entre 3,5 et 10, avantageusement compris entre 5,6 et 7,6. La réaction est, de préférence, effectuée à une température comprise entre 0° et 50°C, avantageusement voisine de 37°C, pendant une période de temps comprise entre 10 secondes et 24 heures. On peut séparer ensuite le matériau biologique du support portant la (ou les) forme(s) de β2GPI, qui a (ou ont) éventuellement fixé au moins une PrP. On effectue éventuellement ensuite un lavage avec une solution, tamponnée de préférence.

Les mêmes conditions de fixation de (ou des) forme(s) de β2GPI sur le support et de fixation de la (ou des) PrP sur la (ou les) forme(s) de β2GPI que celles décrites précédemment peuvent être utilisées lorsque la fixation de la (ou des) forme(s) de β2GPI sur le support est effectuée après la formation du complexe.

Lorsque le complexe est fixé sur le support par l'intermédiaire de sa partie β2GPI, il est alors possible d'isoler la PrP. L'isolement de la partie PrP du complexe fixé sur le support solide par sa partie β2GPI peut se faire selon n'importe quel procédé d'élution utilisé pour la chromatographie d'affinité, tels que ceux décrits dans "Guide to protein purification. Methods in enzymology", édité par Deutscher M., Academic Press, 1990. On sépare ou élue le matériau biologique du support solide contenant la (ou les) forme(s) de β2GPI à l'aide d'un tampon ayant un pH compris entre 2 et 10,5, ayant une concentration en NaCl comprise entre 0 et 5M, avantageusement avec un tampon glycine-HCl, 0,1 mole/litre, ayant un pH de 2,5.

La détection et/ou l'identification et/ou la quantification des PrP fixées sur la (ou les) forme(s) de β2GPI peuvent se faire par tout moyen connu utilisant la détection et/ou l'identification et/ou la quantification par des anticorps, tel que décrit dans "Current Protocols in Immunology" aux Editions Coligan J., Kruisbeek A., Margulies D., Shevach E et Strober W., Wiley Interscience, 1992. Le terme "anticorps" ci-dessus utilisé se réfère à des anticorps polyclonaux ou monoclonaux. Le terme "anticorps monoclonal" se réfère à une composition d'anticorps consistant en une population homogène d'anticorps ; ce terme n'est pas limité en regard de l'espèce productrice de cet anticorps ni de la source de sa provenance, ni de la manière dont il a été produit. La détection et/ou l'identification et/ou la quantification des PrP fixées sur la (ou les) forme(s) de β2GPI se font, de préférence, à l'aide d'un anticorps reconnaissant spécifiquement des antigènes, de préférence de nature protéique, des PrP. De façon connue, cet anticorps peut être conjugué à un marqueur enzymatique, à de l'or colloïdal, à un traceur radioactif, fluorescent ou luminescent. L'excès d'anticorps peut être éliminé par lavage. On peut ajouter ensuite, de façon connue, dans le cas où l'anticorps est couplé à un marqueur enzymatique, un substrat spécifique de l'enzyme conjuguée à l'anticorps, substrat qui se transforme, dans des conditions fixées, en un produit coloré. La formation dudit composé coloré indique la présence de la PrP et permet son identification ainsi que sa quantification.

La détection et/ou l'identification et/ou la quantification des PrP fixées sur la (ou les) forme(s) de β2GPI peuvent se faire par tout moyen connu utilisant la détection et/ou l'identification et/ou la quantification par des procédés d'infection de cellules ou d'organismes susceptibles à l'infection par des PrP, tel que décrit dans "Fields Virology", Third Edition, Lippincott - Raven Publishers, 1996, ou "Virology Methods Manual", édité par Mahy B., Kangro H., Academic Press, 1996.

Selon un deuxième mode de mise en oeuvre de l'invention, dans le cas où l'on fixe le complexe sur un support, on retient le complexe PrP/β2GPI sur le support par l'intermédiaire de la partie PrP dudit complexe ; ensuite, on détecte la partie β2GPI dudit complexe par tout moyen approprié, avantageusement à l'aide d'anticorps spécifiques de la β2GPI, conjugués notamment à un marqueur enzymatique, à de l'or colloïdal, à un traceur radioactif, fluorescent ou luminescent. La présence native ou le rajout de détergent(s) et/ou de lipide(s) peut aider la fixation de ces anticorps. La fixation de la PrP sur le support peut être réalisée, avant ou après la formation du complexe, dans les mêmes conditions que celles décrites précédemment pour la fixation de β2GPI sur le support, et la fixation de la β2GPI sur la PrP pour former le complexe peut être réalisée dans les mêmes conditions que celles décrites précédemment pour fixer la PrP sur la β2GPI.

Dans une première variante des deux modes de mise en oeuvre précités, on retient indirectement le complexe PrP/β2GPI par l'intermédiaire de la partie β2GPI du complexe, en munissant le support d'un composé se liant à ladite partie de la β2GPI ; ensuite la partie PrP du complexe est détectée comme ci-dessus décrit. Le composé se liant à la β2GPI peut être, par exemple, un anticorps reconnaissant la β2GPI ou une autre protéine, par exemple d'origine virale ou cellulaire, procaryote ou eucaryote, ou un composé biologique, par exemple un acide gras ou un lipide, ou un composé chimique, par exemple le dextran sulfate, l'héparine sulfate ou un détergent, tel que celui connu sous le nom commercial "TRITON X 100".

Dans une deuxième variante, on retient indirectement le complexe PrP/β2GPI par l'intermédiaire de la partie PrP dudit complexe en munissant le support d'un composé se liant à ladite partie PrP du complexe; ensuite on détecte et/ou identifie et/ou quantifie la partie β2GPI dudit complexe comme décrit ci-dessus. Le composé se liant à la PrP peut être, par exemple, un anticorps reconnaissant la PrP ou une autre protéine, par exemple d'origine virale ou cellulaire, procaryote ou eucaryote, ou un composé biologique, par exemple un acide gras ou un lipide, ou un composé chimique, par exemple le dextran sulfate, l'héparine sulfate ou un détergent, tel que celui connu sous le nom commercial "TRITON X 100".

Dans ces deux variantes, la fixation indirecte de la (ou des) forme(s) de β2GPI ou de la (ou des) PrP sur le support par l'intermédiaire d'un composé peut être effectuée, après ou avant la formation du complexe, dans des conditions similaires à celles décrites dans le cas d'une fixation directe.

Selon une autre mise en oeuvre de l'invention, on utilise la (ou les) forme(s) de β2GPI naturellement présente(s) dans un matériau biologique. Dans ce cas, on se propose de détecter et/ou d'identifier et/ou de quantifier des PrP lorsque ces PrP sont en quantité telle, par rapport à la β2GPI naturellement présente dans le matériau biologique, qu'elles sont majoritairement complexées ou complexables à au moins une des formes de β2GPI naturellement présentes. Dans ce cas, les formes de β2GPI comprennent toutes formes de β2GPI animale ou humaine naturellement présentes dans le matériau biologique. Le complexe naturellement formé dans le matériau biologique peut alors être fixé sur un support, directement ou indirectement, soit par sa partie PrP, soit par sa partie β2GPI, la détection et/ou identification et/ou quantification du complexe étant réalisée par la partie du complexe non liée directement ou indirectement au support. Ce procédé peut permettre de détecter éventuellement un état initial de la pathologie, alors que le procédé selon la première mise en oeuvre est plus approprié à l'étude d'un état de pathologie déclarée correspondant.

La description donnée ci-après, à titre d'exemples purement illustratifs et non limitatifs, permettra de mieux comprendre l'invention. Les exemples sont décrits en se référant au dessin annexé sur lequel :
- la figure 1 représente les résultats obtenus dans l'exemple 1 ;
- la figure 2 représente les résultats obtenus dans l'exemple 2 ; et
- la figure 3 représente les résultats obtenus dans l'exemple 4.

### Obtention des formes de β2GPI

On utilise, comme matière première de départ, un plasma ou un sérum d'origine humaine ou animale ou une protéine recombinante. Le plasma ou sérum animal est d'origine soit bovine, soit porcine, soit ovine.

La purification des formes de β2GPI est réalisée soit selon le procédé décrit dans le brevet français 2 701 263 soit selon des procédés déjà décrits dans la littérature, notamment "Gambino R, Ruiu G, Pagano G, Cassader M. Chem Phys Lipids, 1999 ; 103(1-2) : 161-74", "Regnault V, Arvieux J, Vallar L, Lecompte T. J Immunol Methods, 1998 ; 211(1-2):191-7", "Klaerke DA, Rojkjaer R, Christensen L, Schousboe I. Biochim Biophys Acta, 1997 ; 1339(2) : 203-16", "Cai G, Guo Y, Shi J. Protein Expr Purif, 1996 ; 8(3) : 341-6", "Gambino R, Ruiu G, Cassader M, Pagano G. J Lipid Res, 1996 ; 37(4) : 902-4", "Williams SC, Sim RB. J Immunol Methods, 1993 ; 157(1-2) : 25-30", "McNeil HP, Simpson RJ, Chesterman CN, Krilis SA. Proc Natl Acad Sci U S A 1990 ; 87(11) : 4120-4", "Lozier J, Takahashi N, Putnam FW. Proc Natl Acad Sci U S A, 1984 ; 81(12) : 3640-4", ou "Lambin P, Burstein M. Biochimie, 1982;64(11-12) : 1065-71".

La séquence des 20 premiers acides aminés de la région N-terminale des formes de β2GPI obtenues après purification ou après purification et fragmentation en polypeptides à l'aide d'enzymes protéolytiques a été déterminée par microséquençage à l'aide d'un appareil "Applied Biosystems Inc, modèle 470" couplé à un analyseur de phénylrhéohydantoïne modèle 120 A (ABI). Les séquences obtenues correspondent avec celles décrites dans la littérature et trouvées dans les banques de données.

Pour des raisons de commodité les formes de β2GPI seront ci-après désignées par :
- **β2GPI N** : β2GPI native dont la séquence correspond à celle publiée par "T. KRISTENSEN et coll., FEBS Letters, Vol. 289, 1991, pages 183-186",
- **β'2GPI :** β2GPI dont la séquence a été donnée dans le brevet français 2 701 263. Cette forme de β2GPI porte la substitution Thr318Ser ;
- **β'2GPI carb. :** β'2GPI dont les résidus lysine ont été modifiés par carbamylation avec du cyanate de potassium à pH 5,8 pendant 4 heures à 37°C selon le procédé décrit par Means GE et Feeney RE, dans "Chemical modification of proteins, Holden-Day Inc, San Francisco, 1971: 215-216" ;
- **β2GPI Bov :** β2GPI purifiée à partir de sérum bovin ;
- **β2GPI Por :** β2GPI purifiée à partir de plasma de porc ;
- **β2GPI Rec :** β2GPI recombinante produite chez des cellules d'insectes après infection avec du baculovirus servant de vecteur du gène de la β2GPI N ;
- **β2GPI Pep1 :** peptide correspondant à la séquence CKNEKKC de la β2GPI et obtenu par synthèse chimique. Les deux cystéines sont reliées par un pont disulfure ;
- **β2GPI Pep2 :** peptide correspondant à la séquence CKNEKKC de la β2GPI et obtenu par synthèse chimique. Les deux cystéines sont libres.

### EXEMPLE 1

Des préparations tissulaires d'animaux infectés par l'agent de l'encéphalite spongiforme bovine (ESB) ont été réalisées comme décrit dans "Maignien T. et coll., Journal of General Virology, 1999, 80 : 3035-3042".

La PrP^{SC} (protéine du prion responsable de la transmission de l'encéphalite spongiforme) a été purifiée, selon la référence précédente, par centrifugation en présence de détergents, après digestion par la protéinase K. Lors de ce traitement, la PrP^{C} est détruite par la protéinase K et le(s) détergent(s). Les échantillons purifiés ont par la suite été séparés sur un gel d'éléctrophorèse à 12% de polyacrylamide, en présence de sodium dodécyl sulfate (SDS), puis transférés sur une membrane de nitrocellulose (Schleicher & Schuell). La membrane a ensuite été saturée avec de l'albumine bovine à 2%, pendant 1 heure à température ambiante puis découpée en plusieurs bandelettes.

De la β'2GPI, β2GPI N, β2GPI carb. et β'2GPI Rec, couplées à de la phosphatase alcaline, ont été déposées sur ces bandelettes (essais 1, 3, 5, 7 respectivement) et, à titre de comparaison, sur des bandelettes obtenues à partir de préparations tissulaires d'animaux non-infectés par l'agent de l'encéphalite spongiforme bovine (ESB) (essais 2, 4, 6, 8 respectivement).

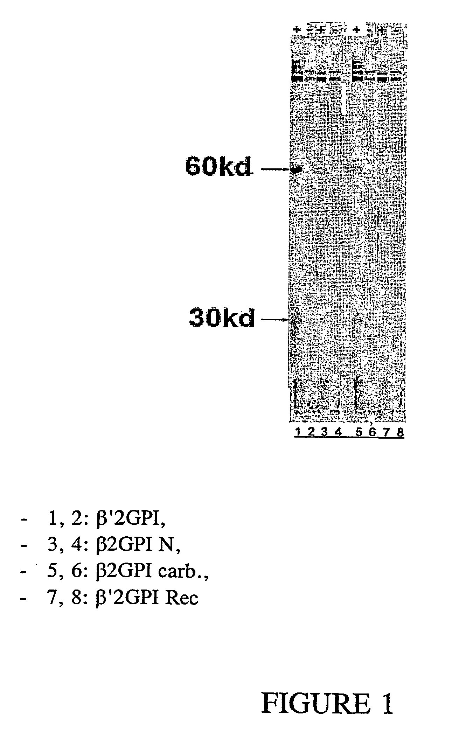

L'expérience montrée par la figure 1 a été réalisée comme suit : les bandelettes ont été rincées trois fois avec du tampon acétate 50 mM, pH 5,6, Triton X100 0,05%. Un millilitre à 1 µg/ml des différentes formes de β2GPI, dans du tampon acétate 50 mM, pH 5,6, gélatine 0,1%, Triton X 100 0,5%, a été ajouté sur chaque bandelette. Après une incubation d'une heure à température ambiante et sous agitation, les bandelettes ont été lavées 6 fois avec du PBS (tampon phosphate salin) contenant 0,05% de Triton X 100. La révélation des formes de β2GPI fixées a été réalisée avec un mélange liquide de substrat 5-bromo-4-chloro-3-indolyl phosphate / nitro blue tetrazolium (BCIP/NBT).

La figure 1 montre qu'avec les différentes formes de β2GPI, il est possible de détecter les PrP^{SC} dans le cas d'animaux infectés par l'agent de l'encéphalite spongiforme bovine (ESB).

### EXEMPLE 2

De la protéine bovine recombinante PrP a été obtenue de la Société "Prionics". Le support solide utilisé est une plaque de microtitration de type "C8 Starwell Maxisorp" à 96 puits et à fond plat, commercialisée par la société "NUNC". L'expérience de fixation sur support solide sur lequel une des formes de β2GPI a été fixée a été réalisée comme suit. La PrP bovine recombinante a été utilisée sur une gamme de concentrations allant de 0 à 2000 ng/puits. La dilution est effectuée à l'aide d'un tampon (Tris/HCl) ayant une concentration en Tris de 0,05 mole/l et un pH de 7,6 ± 0,05. On dépose 100 µl de solution au fond de chaque puits de la plaque. La plaque est incubée à +37°C pendant une période de 90 minutes. Après cette incubation, on effectue un lavage en introduisant 300 µl de tampon phosphate dans chaque puits, on laisse en contact pendant 2 minutes et on aspire la solution de tampon ; on renouvelle cette opération de lavage 4 fois.

La révélation de la PrP fixée sur les différentes formes de β2GPI a été effectuée en utilisant une solution d'anticorps monoclonal 6H4, obtenue de la Société "Prionics". Cet anticorps monoclonal 6H4 est spécifique à la PrP.

On ajoute par puits 100 µl d'une solution d'anticorps monoclonal 6H4 dilué 5000 fois en tampon phosphate salin (PBS). On laisse la plaque incuber à 37°C pendant 60 minutes. A la suite de cette incubation, le contenu des puits de la plaque est aspiré. On introduit 300 µl de tampon phosphate dans chaque puits et après un temps de contact de 2 minutes, on aspire le tampon : cette opération de lavage est renouvelée 4 fois.

On ajoute par puits 100 µl d'une solution d'anticorps de lapin spécifique des IgG de souris, conjugué à la péroxidase. On laisse la plaque incuber à 37°C pendant 60 minutes. A la suite de cette incubation, le contenu des puits de la plaque est aspiré. On introduit 300 µl de tampon phosphate dans chaque puits et après un temps de contact de 2 minutes, on aspire le tampon : cette opération de lavage est renouvelée 6 fois.

On ajoute par puits 100 µl d'une solution d'o-phénylène-diamine, 2HCl dans un tampon citrate de sodium. On laisse incuber pendant 30 minutes à température ambiante, puis on arrête la réaction en rajoutant à chaque puits 50 µl de H₂SO₄, 2N. On mesure l'absorbance à 492 nm obtenue en fin de réaction à l'aide d'un robot lecteur de plaque.

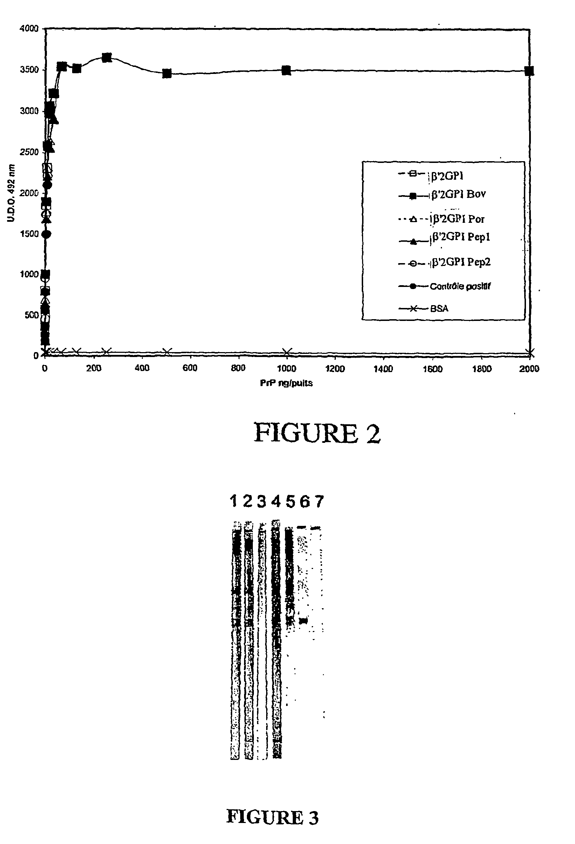

Le tableau 1 et la figure 2 correspondante montrent que la PrP recombinante, dont la séquence est celle des deux formes de protéine prion PrP^{C} et PrP^{SC}, peut être détectée avec les différentes formes de β2GPI. Le contrôle positif est de la PrP recombinante fixée sur support solide aux différentes concentrations notées dans le tableau 1 puis révélée avec la solution d'anticorps monoclonal 6H4. La BSA (albumine sérique bovine) sert de contrôle négatif démontrant que la liaison est spécifique aux formes de β2GPI.

**TABLEAU 1**

| Valeurs de densité optique (UDO x 1000) | | | | | | | |
|---|---|---|---|---|---|---|---|
| Concentration | β'2GPI | β2GPI | β2GPI | β2GPI | β2GPI | Témoin | BSA |
| PrP | | Bov. | Por. | Pep1 | Pep2 | Positif | |
| ng/puits | | | | | | | |
| 2000 | 3500 | 3500 | 3500 | 3500 | 3500 | 3500 | 42 |
| 1000 | 3500 | 3500 | 3500 | 3500 | 3500 | 3500 | 42 |
| 500 | 3458 | 3458 | 3458 | 3458 | 3458 | 3458 | 42 |
| 250 | 3648 | 3648 | 3648 | 3648 | 3648 | 3648 | 43 |
| 125 | 3521 | 3521 | 3521 | 3521 | 3521 | 3521 | 43 |
| 62.5 | 3542 | 3542 | 3542 | 3542 | 3542 | 3542 | 42 |
| 31.25 | 3215 | 3215 | 2915 | 2904 | 3215 | 3215 | 45 |
| 15.62 | 2985 | 3060 | 2650 | 2558 | 2965 | 2965 | 42 |
| 7.812 | 2315 | 2580 | 2310 | 2214 | 2212 | 2105 | 43 |
| 3.91 | 1856 | 1896 | 1782 | 1695 | 1746 | 1500 | 44 |
| 1.95 | 798 | 1005 | 698 | 649 | 956 | 785 | 43 |
| 0.98 | 463 | 564 | 369 | 322 | 452 | 256 | 42 |
| 0.49 | 286 | 365 | 195 | 188 | 298 | 185 | 42 |

### EXEMPLE 3

De la protéine bovine recombinante PrP, identique à celle utilisée dans l'exemple 2, a été ajoutée dans du sérum humain.

Le support solide utilisé est une plaque de microtitration de type "C8 Starwell Maxisorp" à 96 puits et à fond plat, commercialisée par la société "NUNC". Diverses solutions de composés ont été fixées sur le support, à savoir une solution de protéines recombinantes p26-HIV2 ROD reconnaissant la β2GPI, des anticorps monoclonaux reconnaissant la βGPI, du dextran sulfate, de l'héparine sulfate reconnaissant la protéine prion PrP ainsi que la β2GPI, et des anticorps monoclonaux reconnaissant la protéine prion PrP. La β2GPI utilisée dans cet exemple est la β2'GPI. Le sérum contenant la PrP bovine recombinante a été dilué 50 fois. La dilution est effectuée à l'aide d'un tampon (Tris/HCl) ayant une concentration en Tris de 0,05 mole/l et un pH de 7,6 ± 0,05. On dépose 100 µl de solution au fond de chaque puits de la plaque. La plaque est incubée à +37°C pendant une période de 90 minutes. Après cette incubation, on effectue un lavage en introduisant 300 µl de tampon phosphate dans chaque puits, on laisse en contact pendant 2 minutes et on aspire la solution de tampon ; on renouvelle cette opération de lavage 4 fois.

La révélation de la partie PrP du complexe a été effectuée en utilisant une solution d'anticorps monoclonal 6H4, obtenue de la Société "Prionics". La révélation de la partie β2GPI du complexe a été effectuée en utilisant une solution d'anticorps monoclonal 8C3.

On ajoute par puits 100 µl d'une solution d'anticorps monoclonal 6H4 ou 8C3 dilué 5000 fois en tampon phosphate salin (PBS). On laisse la plaque incuber à 37°C pendant 60 minutes. A la suite de cette incubation, le contenu des puits de la plaque est aspiré. On introduit 300 µl de tampon phosphate dans chaque puits et après un temps de contact de 2 minutes, on aspire le tampon : cette opération de lavage est renouvelée 4 fois.

On ajoute par puits 100 µl d'une solution d'anticorps monoclonal de souris spécifique des IgG de souris conjugué à la péroxidase. On laisse la plaque incuber à 37°C pendant 60 minutes. A la suite de cette incubation, le contenu des puits de la plaque est aspiré. On introduit 300 µl de tampon phosphate dans chaque puits et après un temps de contact de 2 minutes, on aspire le tampon : cette opération de lavage est renouvelée 6 fois.

On ajoute par puits 100 µl d'une solution d'o-phénylène-diamine, 2HC1 dans un tampon citrate de sodium. On laisse incuber pendant 30 minutes à température ambiante, puis on arrête la réaction en rajoutant dans chaque puits 50 µl de H₂SO₄, 2N. On mesure l'absorbance à 492 nm obtenue en fin de réaction à l'aide d'un robot lecteur de plaque.

La moyenne des absorbances obtenues pour chaque solution de composé fixée sur le support est donnée par le tableau 2 (en unités de densité optique multipliées par 1000). Les résultats du tableau 2 démontrent que le complexe PrP/β2GPI peut être détecté selon le mode de la présente invention, et, en particulier, que la PrP recombinante peut être détectée.

**TABLEAU 2**

| Valeurs de densité optique (UDO x 1000) | | |
|---|---|---|
| Composé fixé sur le | Révélation de la partie | Révélation de la partie |
| support | β2GPI du complexe | PrP du complexe |
| Héparine sulfate | 1896 | 1756 |
| P26-HIV2 ROD | - | 1615 |
| Dextran sulfate | 1752 | 1459 |
| Anticorps anti-β2GPI⁽¹⁾ | - | 1763 |
| Anticorps anti-PrP^{SC(2)} | 1862 | - |
| ⁽¹⁾ anticorps monoclonaux 8C3 | | |
| ⁽²⁾ anticorps monoclonaux 6H4 | | |

### EXEMPLE 4

Les protéines d'un sérum provenant d'un sujet sain ont été séparées sur un gel d'électrophorèse à 12 % de polyacrylamide, en présence de sodium dodécyl sulfate (SDS), puis transférées sur une membrane de nitrocellulose (Schleicher & Schuell). La membrane a été saturée avec du lait écrémé à 2 % en PBS (tampon phosphate salin), pendant 1 heure à température ambiante puis découpée en plusieurs bandelettes. De la protéine bovine recombinante PrP, identique à celle utilisée dans l'exemple 2, a été déposée de la manière suivante sur des bandelettes numérotées de 1 à 5 :
- 1 µg de protéine bovine recombinante PrP, dans du PBS, a été déposé sur la bandelette 1 ;
- 1 µg de protéine bovine recombinante PrP, dans du Tris 0,05 mole/l, pH 7,6, NaCl 0,15 mole/l, a été déposé sur les bandelettes 2 et 5 ;
- 1 µg de protéine bovine recombinante PrP, dans du Tris 0,05 mole/l, pH 7,6, NaCl 0,15 mole/l, lysine 0,1 mole/l, a été déposé sur la bandelette 4 ;
- 1 µg de protéine bovine recombinante PrP, dans du Tris 0,05 mole/l, pH 7,6, NaCl 0,15 mole/l, a été déposé sur la bandelette 3, qui avait au préalable été préincubée pendant 1 heure avec un anticorps monoclonal, le 8C3, dirigé contre la β2GPI.

Après 1 heure d'incubation, sous agitation et à température ambiante, les bandelettes 1 à 5 ont été rincées 4 fois avec du PBS. Une solution d'anticorps monoclonal 6H4, dirigé contre la PrP, diluée 5 000 fois en tampon Tris HCl 0,05 mole/l, pH 7,6, NaCl 0,15 mole/l, gélatine 0,2 %, Tween 20 0,05 %, a été ajoutée aux bandelettes.

Une solution d'anticorps monoclonal 8C3 dirigé contre la β2GPI, contenant 1 µg d'anticorps/ml en tampon Tris HCl 0,05 ml/l, pH 7,6, NaCl 0,15 mole/l, gélatine 0,2 %, Tween 20 0,05 %, a été ajoutée sur une bandelette 6.

Après 1 heure d'incubation, sous agitation et à température ambiante, les bandelettes 1 à 6 ont été rincées quatre fois avec du PBS.

Finalement, les bandelettes 1 à 6, ainsi qu'une bandelette 7 destinée à servir de témoin négatif, ont été incubées pendant 1 heure, à température ambiante et sous agitation, en présence d'une solution d'anticorps de lapin couplés à la phosphatase alcaline et dirigés contre les anticorps de souris, dans du tampon Tris HCl 0,05 mole/l, pH 7,6, NaCl 0,2 mole/l, gélatine 0,2 %, Tween 20 0,05 %, puis rincées 6 fois avec du tampon PBS contenant 0,05 % de Tween 20. La révélation des anticorps a été réalisée avec un mélange liquide de substrat 5-bromo-4-chloro-3-indolyl phosphate/nitro blue tetrazolium (BCIP/NBT). Les résultats obtenus sont présentés à la figure 3.

La bandelette 6 de la figure 3 montre l'emplacement de la β2GPI N avec une grosse tache au niveau des monomères et des taches plus faibles au niveau des dimères et polymères.

Les bandelettes 1, 2 et 5 montrent les protéines sériques liant la PrP recombinante. Ces protéines sont situées au même niveau que celles reconnues par l'anticorps monoclonal dirigé contre la β2GPI.

La bandelette 3 montre que les protéines liant la PrP recombinante sont celles qui sont reconnues par l'anticorps monoclonal dirigé contre la β2GPI (absence de signal après blocage de ces protéines).

Cette expérience montre que, parmi les protéines sériques, la β2GPI N reconnaît et fixe la PrP recombinante.

## Revendications

1. Procédé de séparation et/ou de détection et/ou d'identification et/ou de quantification dans un matériau biologique d'au moins une protéine prion PrP, **caractérisé par le fait qu'**il comprend une étape de séparation et/ou de détection et/ou d'identification et/ou de quantification d'un complexe PrP/β2GPI formé d'au moins une protéine prion liée à au moins une forme de β2-glycoprotéine I désignée en abrégé par β2GPI.

2. Procédé selon la revendication 1, **caractérisé par le fait qu'**il comprend une étape de séparation et/ou de détection et/ou d'identification et/ou de quantification d'un complexe PrP^{SC}/β2GPI formé d'au moins une protéine prion anormale PrP^{SC} liée à au moins une forme de β2GPI, ledit procédé constituant un procédé de séparation et/ou de détection et/ou d'identification et/ou de quantification dans un matériau biologique d'au moins une protéine prion anormale.

3. Procédé selon la revendication 2, **caractérisé par le fait que**, pour former un complexe PrP^{SC}/β2GPI, on utilise au moins une protéine prion anormale PrP^{SC} provenant de la tremblante du mouton ou de la chèvre, de l'encéphalopathie bovine, de la maladie du dépérissement chronique des ruminants sauvages, des encéphalopathies des visons ou des chats, de la maladie de Creutzfeld-Jacob, du syndrome de Gerstmann-Straüssler-Scheinker, du kuru ou de l'insomnie familiale fatale.

4. Procédé selon l'une des revendications 2 ou 3, **caractérisé par le fait que**, préalablement à l'étape de séparation et/ou de détection et/ou d'identification et/ou de quantification du complexe PrP^{SC}/β2GPI, on soumet le matériau biologique à l'action de détergents et/ou d'enzymes.

5. Procédé selon la revendication 4, **caractérisé par le fait que**, préalablement à l'étape de séparation et/ou de détection et/ou d'identification et/ou de quantification du complexe PrP^{SC}/β2GPI, on soumet le matériau biologique à l'action de la protéinase K.

6. Procédé selon l'une des revendications 1 à 5, **caractérisé par le fait que** l'on utilise, pour former un complexe PrP/β2GPI, au moins une β2GPI d'origine humaine, ou d'origine animale, une β2GPI recombinante, ou une β2GPI obtenue par synthèse chimique ou une forme modifiée de β2GPI.

7. Procédé selon l'une des revendications 1 à 6, **caractérisé par le fait que** l'on réalise une étape de fixation de PrP contenue dans un matériau biologique à au moins une forme de β2GPI intentionnellement rajoutée audit matériau biologique pour former ledit complexe, suivie d'une étape de séparation et/ou de détection et/ou d'identification et/ou de quantification du complexe PrP/β2GPI.

8. Procédé selon la revendication 7, **caractérisé par le fait que** l'on réalise :
- une étape de fixation sur un support, d'au moins une forme de β2GPI ou de ladite (ou desdites) PrP, avant ou après l'étape de fixation de ladite (ou desdites) PrP à ladite (ou auxdites) forme(s) de β2GPI pour former ledit complexe,
- une étape de séparation consistant à séparer le matériau biologique du support, sur lequel est fixé le complexe,
- une étape de détection et/ou d'identification et/ou de quantification consistant, après ladite étape de séparation, à détecter et/ou à identifier et/ou à quantifier le complexe fixé au support par sa partie, qui n'est pas liée au support.

9. Procédé selon la revendication 8, **caractérisé par le fait que** l'on utilise, comme support, un support solide.

10. Procédé selon l'une des revendications 8 ou 9, **caractérisé par le fait que** l'on effectue la fixation sur le support par l'intermédiaire d'un composé se liant à l'une des parties PrP ou β2GPI du complexe, ladite étape de détection et/ou d'identification et/ou de quantification consistant à détecter et/ou à identifier et/ou à quantifier le complexe par sa partie, qui n'est pas liée au support.

11. Procédé selon la revendication 10, **caractérisé par le fait que** le composé se liant à la β2GPI ou à la PrP est un anticorps reconnaissant respectivement la β2GPI ou la PrP, ou bien une autre protéine, un composé biologique, un composé chimique ou un détergent se fixant à la PrP ou à la β2GPI.

12. Procédé selon l'une des revendications 8 à 11, **caractérisé par le fait que** l'on réalise :
- l'étape de fixation d'au moins une forme de β2GPI ou de la (ou des) PrP sur un support, par réaction de groupes réactifs de la (ou des) forme(s) de β2GPI ou de la (ou des) PrP avec des sites réactifs du support, ladite (ou lesdites) forme(s) étant mise(s) en solution dans un tampon ayant un pH compris entre 2,5 et 10,5, de préférence entre 5,5 et 7,5, pour obtenir une solution ayant une concentration comprise entre 0,01 et 100g/l de forme(s) de β2GPI ou de PrP, le support étant maintenu en contact avec la solution à une température comprise entre 0° et 40°C pendant un temps d'incubation compris entre 10 secondes et 24 heures, puis
- la séparation du support et de la solution par lavage du support.

13. Procédé selon l'une des revendications 7 à 12, **caractérisé par le fait que** l'étape de fixation d'au moins une PrP à au moins une forme de β2GPI pour former un complexe est réalisée par mise en contact d'au moins une forme de β2GPI, avec le matériau biologique susceptible de contenir des PrP à une température comprise entre 0° et 50°C, avantageusement voisine de 37°C, pendant une période de temps comprise entre 10 secondes et 24 heures, le matériau biologique étant dilué à l'aide d'un tampon donnant un pH compris entre 3,5 et 10, de préférence compris entre 5,6 et 7,6.

14. Procédé selon l'une des revendications 7 à 13, **caractérisé par le fait que** l'on effectue la détection et/ou l'identification et/ou la quantification de la (ou des) PrP du complexe par des anticorps spécifiques de la (ou des) PrP, ou par des procédés d'infection de cellules ou d'organismes susceptibles à l'infection des PrP.

15. Procédé selon la revendication 14, **caractérisé par le fait que** l'on effectue la détection et/ou l'identification et/ou la quantification de la (ou des) PrP du complexe par un anticorps reconnaissant spécifiquement un antigène, de préférence de nature protéique, de la (ou des) PrP.

16. Procédé selon l'une des revendications 7 à 13, **caractérisé par le fait que** l'on effectue la détection et/ou l'identification et/ou la quantification de la β2GPI du complexe par des anticorps spécifiques de la β2GPI.

17. Procédé selon l'une des revendications 14 à 16, **caractérisé par le fait que** l'on couple l'anticorps à un marqueur enzymatique, à de l'or colloïdal, à un traceur radioactif, fluorescent ou luminescent.

18. Procédé selon la revendication 17, **caractérisé par le fait que** l'on couple l'anticorps à un marqueur enzymatique, dont l'enzyme est mise en contact à un substrat spécifique apte à se transformer en un produit coloré.

19. Procédé selon l'une des revendications 8 à 15, **caractérisé par le fait que** l'on fixe le complexe au support par l'intermédiaire de sa partie β2GPI, l'étape de séparation comprenant l'isolement de la PrP du complexe fixé au support par un procédé d'élution de chromatographie d'affinité.

20. Procédé selon la revendication 19, **caractérisé par le fait que** ledit isolement est réalisé par élution de la PrP fixée au support solide à l'aide d'un tampon ayant un pH compris entre 2 et 10,5, une concentration en NaCl comprise entre 0 et 5M, de préférence à l'aide d'un tampon glycine-HCl 0,1 mole/litre ayant un pH de 2,5.

21. Procédé selon l'une des revendications 1 à 6, de séparation et/ou de détection et/ou d'identification et/ou de quantification d'au moins une PrP dans un matériau biologique qui contient naturellement au moins une forme de β2GPI, **caractérisé par le fait qu'**il comprend une étape de séparation et/ou de détection et/ou d'identification et/ou de quantification d'un complexe PrP/β2GPI formé d'au moins une PrP liée à au moins une forme de β2GPI naturellement présente dans ledit matériau.

## Claims

1. A method for separating and/or detecting and/or identifying and/or quantifying, in a biological material, at least one prion protein PrP, **characterized in that** it comprises a step of separating and/or detecting and/or identifying and/or quantifying a complex PrP/β2GPI made up of at least one prion protein bound to at least one form of β2-glycoprotein I called in short β2GPI.

2. The method as claimed in claim 1, **characterized in that** it comprises a step of separating and/or detecting and/or identifying and/or quantifying a complex PrP^{SC}/β2GPI made up of at least one abnormal prion protein PrP^{SC} bound to at least one form of β2GPI, said method constituting a method for separating and/or detecting and/or identifying and/or quantifying, in a biological material, at least one abnormal prion protein.

3. The method as claimed in claim 2, **characterized in that**, to form a complex PrP^{SC}/β2GPI, use is made of at least one abnormal prion protein PrP^{SC} originating from scrapie in sheep or goats, bovine encephalopathy, chronic wasting disease in wild ruminants, mink or cat encephalopathies, Creutzfeld-Jacob disease, Gerstmann-Straussler-Scheinker syndrome, kuru or fatal familial insomnia.

4. The method as claimed in either of claims 2 and 3, **characterized in that**, prior to the step of separating and/or detecting and/or identifying and/or quantifying the complex PrP^{SC}/β2GPI, the biological material is subjected to the action of detergents and/or of enzymes.

5. The method as claimed in claim 4, **characterized in that**, prior to the step of separating and/or detecting and/or identifying and/or quantifying the complex PrP^{SC}/β2GPI, the biological material is subjected to the action of proteinase K.

6. The method as claimed in one of claims 1 to 5, **characterized in that**, to form a complex PrP/β2GPI, use is made of at least one β2GPI of human origin or of animal origin, a recombinant β2GPI, a β2GPI obtained by chemical synthesis, or a modified form of β2GPI.

7. The method as claimed in one of claims 1 to 6, **characterized in that** a step of attaching PrP contained in a biological material to at least one form of β2GPI intentionally added to said biological material is carried out so as to form said complex, followed by a step of separating and/or detecting and/or identifying and/or quantifying the complex PrP/β2GPI.

8. The method as claimed in claim 7, **characterized in that** the following are carried out:
- a step of attaching to a support at least one form of β2GPI or said PrP(s), before or after the step of attaching said PrP(s) to said form(s) of β2GPI so as to form said complex,
- a separation step consisting in separating the biological material from the support to which the complex is attached,
- a step of detection and/or identification and/or quantification consisting, after said separation step, in detecting and/or identifying and/or quantifying the complex attached to the support, via its component which is not bound to the support.

9. The method as claimed in claim 8, **characterized in that** a solid support is used as support.

10. The method as claimed in either of claims 8 and 9, **characterized in that** the attachment to the support is carried out by virtue of a compound which binds to one of the PrP or β2GPI components of the complex, said step of detection and/or identification and/or quantification consisting in detecting and/or identifying and/or quantifying the complex via its component which is not bound to the support.

11. The method as claimed in claim 10, **characterized in that** the compound which binds to the β2GPI or to the PrP is an antibody which recognizes respectively the β2GPI or the PrP, or else another protein, a biological compound, a chemical compound or a detergent which attaches to the PrP or to the β2GPI.

12. The method as claimed in one of claims 8 to 11, **characterized in that**:
- the step of attaching at least one form of β2GPI or the PrP(s) to a support is carried out by reacting reactive groups of the form(s) of β2GPI or of the PrP(s) with reactive sites of the support, said form(s) being dissolved in a buffer having a pH of between 2.5 and 10.5, preferably between 5.5 and 7.5, so as to obtain a solution having a concentration of between 0.01 and 100 g/l of form(s) of β2GPI or of PrP, the support being kept in contact with the solution at a temperature of between 0° and 40°C for an incubation period of between 10 seconds and 24 hours, and then
- the separation of the support and the solution is carried out by washing the support.

13. The method as claimed in one of claims 7 to 12, **characterized in that** the step of attaching at least one PrP to at least one form of β2GPI so as to form a complex is carried out by bringing at least one form of β2GPI into contact with the biological material liable to contain PrPs at a temperature of between 0° and 50°C, advantageously in the region of 37°C, for a period of time of between 10 seconds and 24 hours, the biological material being diluted using a buffer giving a pH of between 3.5 and 10, preferably between 5.6 and 7.6.

14. The method as claimed in one of claims 7 to 13, **characterized in that** the detection and/or identification and/or quantification of the PrP(s) of the complex are carried out using PrP-specific antibodies, or using methods for infecting cells or organisms susceptible to PrP infection.

15. The method as claimed in claim 14, **characterized in that** the detection and/or identification and/or quantification of the PrP(s) of the complex are carried out using an antibody which specifically recognizes an antigen, preferably protein in nature, of the PrP(s).

16. The method as claimed in one of claims 7 to 13, **characterized in that** the detection and/or identification and/or quantification of the β2GPI of the complex are carried out using β2GPI-specific antibodies.

17. The method as claimed in one of claims 14 to 16, **characterized in that** the antibody is coupled to an enzyme label, to colloidal gold, or to a radioactive, fluorescent or luminescent tracer.

18. The method as claimed in claim 17, **characterized in that** the antibody is coupled to an enzyme label for which the enzyme is brought into contact with a specific substrate able to be converted into a colored product.

19. The method as claimed in one of claims 8 to 15, **characterized in that** the complex is attached to the support by virtue of its β2GPI component, the separation step comprising isolating the PrP of the complex attached to the support by an affinity chromatography elution method.

20. The method as claimed in claim 19, **characterized in that** said isolation is carried out by elution of the PrP attached to the solid support using a buffer having a pH of between 2 and 10.5 and an NaCl concentration of between 0 and 5M, preferably using a 0.1 mol/liter glycine-HCl buffer having a pH of 2.5.

21. The method as claimed in one of claims 1 to 6, for separating and/or detecting and/or identifying and/or quantifying at least one PrP in a biological material which naturally contains at least one form of β2GPI, **characterized in that** it comprises a step of separating and/or detecting and/or identifying and/or quantifying a complex PrP/β2GPI made up of at least one PrP bound to at least one form of β2GPI naturally present in said material.

## Patentansprüche

1. Verfahren zur Abtrennung und/oder Detektion und/oder Identifikation und/oder Quantifikation mindestens eines Prionenproteins PrP in biologischem Material, **dadurch gekennzeichnet, dass** es einen Schritt der Abtrennung und/oder Detektion und/oder Identifikation und/oder Quantifikation eines PrP/β2GPI-Komplexes umfasst, gebildet aus mindestens einem an mindestens eine Form des β2-Glykoproteins I gebundenen Prionenprotein, abgekürzt als β2GPI bezeichnet.

2. Verfahren nach Anspruch 1, **dadurch gekennzeichnet, dass** es einen Schritt der Abtrennung und/oder Detektion und/oder Identifikation und/oder Quantifikation eines PrP^{SC}/ β2GPI-Komplexes umfasst, gebildet aus mindestens einem an mindestens eine Form von β2GPI gebundenen anomalen Prionenprotein PrP^{SC}, wobei das Verfahren ein Verfahren zur Abtrennung und/oder Detektion und/oder Identifikation und/oder Quantifikation von mindestens einem anomalen Prionenprotein in biologischem Material ist.

3. Verfahren nach Anspruch 2, **dadurch gekennzeichnet, dass** man zur Bildung eines PrP^{SC}/β2GPI-Komplexes mindestens ein anomales Prionenprotein PrP^{SC} verwendet, das von der Traberkrankheit der Schafe oder Ziegen (Scrapie), der Rinderenzephalopathie, dem chronischen Siechtum der wilden Wiederkäuer, den Enzephalopathien der Nerze oder der Katzen, der Creutzfeldt-Jakob-Krankheit, dem Gerstmann-Sträussler-Scheinker-Syndrom, dem Kuru oder der tödlichen familiären Schlaflosigkeit stammt.

4. Verfahren nach einem der Ansprüche 2 oder 3, **dadurch gekennzeichnet, dass** man vor dem Schritt der Abtrennung und/oder Detektion und/oder Identifikation und/oder Quantifikation des PrP^{SC}/β2GPI-Komplexes das biologische Material der Einwirkung von Detergenzien und/oder Enzymen unterzieht.

5. Verfahren nach Anspruch 4, **dadurch gekennzeichnet, dass** man vor dem Schritt der Abtrennung und/oder Detektion und/oder Identifikation und/oder Quantifikation des PrP^{SC}/β2GPI-Komplexes das biologische Material der Einwirkung von Proteinase K unterzieht.

6. Verfahren nach einem der Ansprüche 1 bis 5, **dadurch gekennzeichnet, dass** man zur Bildung eines PrP^{SC}/β2GPI-Komplexes mindestens ein β2GPI menschlichen Ursprungs, tierischen Ursprungs, ein rekombinantes β2GPI, ein durch chemische Synthese erhaltenes β2GPI oder eine modifizierte Form von β2GPI verwendet.

7. Verfahren nach einem der Ansprüche 1 bis 6, **dadurch gekennzeichnet, dass** man einen Schritt der Fixierung des in einem biologischen Material enthaltenen PrP an mindestens eine diesem biologischen Material gezielt zugesetzte Form von β2GPI zur Bildung des Komplexes durchführt, gefolgt von einem Schritt der Abtrennung und/oder Detektion und/oder Identifikation und/oder Quantifikation des PrP/β2GPl-Komplexes.

8. Verfahren nach Anspruch 7, **dadurch gekennzeichnet, dass** man:
- einen Schritt der Fixierung von mindestens einer Form von β2GPI oder von PrP an einen Träger, vor oder nach dem Schritt der Fixierung von PrP an diese Form oder Formen von β2GPI zur Bildung des Komplexes,
- einen Abtrennungsschritt, der darin besteht, dass man das biologische Material von dem Träger, an den der Komplex fixiert ist, abtrennt,
- einen Schritt der Detektion und/oder Identifikation und/oder Quantifikation, der darin besteht, dass man nach dem Abtrennungsschritt den an den Träger fixierten Komplex anhand seines nicht an den Träger gebundenen Bestandteils detektiert und/oder identifiziert und/oder quantifiziert,
durchführt.

9. Verfahren nach Anspruch 8, **dadurch gekennzeichnet, dass** man als Träger einen festen Träger verwendet.

10. Verfahren nach einem der Ansprüche 8 oder 9, **dadurch gekennzeichnet, dass** man die Fixierung an den Träger durch Vermittlung einer sich an einen der PrP- oder β2GPI-Teile des Komplexes bindenden Substanz bewerkstelligt, wobei der Schritt der Detektion und/oder Identifikation und/oder Quantifikation darin besteht, dass man den Komplex anhand seines nicht an den Träger gebundenen Teils detektiert und/oder identifiziert und/oder quantifiziert.

11. Verfahren nach Anspruch 10, **dadurch gekennzeichnet, dass** die an β2GPI oder PrP bindende Substanz ein Antikörper ist, der β2GPI bzw. PrP erkennt, oder auch ein anderes Protein, eine biologische Substanz, eine chemische Verbindung oder ein Detergenz, das bzw. die an PrP oder β2GPl bindet.

12. Verfahren nach einem der Ansprüche 8 bis 11, **dadurch gekennzeichnet, dass** man:
- den Schritt der Fixierung mindestens einer Form von β2GPI oder PrP an einen Träger durch Reaktion reaktiver Gruppen der β2GPI-Form oder -Formen oder PrP-Form oder -Formen mit reaktiven Stellen des Trägers, wobei diese Form oder Formen in einem Puffer mit einem pH-Wert von 2,5 bis 10,5, vorzugsweise von 5,5 bis 7,5 gelöst ist/sind, um eine Lösung mit einer Konzentration von 0,01 bis 100 g/l der β2GPI- oder PrP-Form oder -Formen zu erhalten, wobei der Träger bei einer Temperatur von 0 ° bis 40 °C über eine Inkubationszeit von 10 Sekunden bis 24 Stunden im Kontakt mit der Lösung gehalten wird, dann
- die Abtrennung des Trägers und der Lösung durch Waschen des Trägers durchführt.

13. Verfahren nach einem der Ansprüche 7 bis 12, **dadurch gekennzeichnet, dass** der Schritt der Fixierung mindestens einer PrP-Form an mindestens eine β2GPI-Form zur Bildung eines Komplexes **dadurch** durchgeführt wird, dass man mindestens eine β2GPI-Form bei einer Temperatur von 0 ° bis 50 °C, vorzugsweise in der Nähe von 37 °C, über einen Zeitraum von 10 Sekunden bis 24 Stunden hinweg in Kontakt mit dem biologischen Material bringt, das imstande ist, PrP zu enthalten, wobei das biologische Material unter Zuhilfenahme eines Puffers verdünnt ist, der einen pH-Wert von 3,5 bis 10 vermittelt, vorzugsweise von 5,6 bis 7,6.

14. Verfahren nach einem der Ansprüche 7 bis 13, **dadurch gekennzeichnet, dass** man die Detektion und/oder Identifikation und/oder Quantifikation des PrP des Komplexes durch für PrP spezifische Antikörper oder durch Verfahren der Infektion von für Infektion mit PrP empfindlichen Zellen oder Organismen durchführt.

15. Verfahren nach Anspruch 14, **dadurch gekennzeichnet, dass** man die Detektion und/oder Identifikation und/oder Quantifikation des PrP im Komplex durch einen Antikörper bewerkstelligt, der spezifisch ein Antigen, vorzugsweise proteinöser Natur, von PrP erkennt.

16. Verfahren nach einem der Ansprüche 7 bis 13, **dadurch gekennzeichnet, dass** man die Detektion und/oder Identifikation und/oder Quantifikation des β2GPl-Komplexes durch für β2GPI spezifische Antikörper bewerkstelligt.

17. Verfahren nach einem der Ansprüche 14 bis 16, **dadurch gekennzeichnet, dass** man den Antikörper mit einer enzymatischen Markierung, mit kolloidalem Gold oder mit einem radioaktiven, fluoreszierenden oder lumineszenten Tracer koppelt.

18. Verfahren nach Anspruch 17, **dadurch gekennzeichnet, dass** man den Antikörper mit einem enzymatischen Marker koppelt, wobei das Enzym mit einem spezifischen Substrat in Kontakt gebracht wird, das geeignet ist, in ein farbiges Produkt umgewandelt zu werden.

19. Verfahren nach einem der Ansprüche 8 bis 15, **dadurch gekennzeichnet, dass** man den Komplex durch Vermittlung seines β2GPI-Teils an den Träger fixiert, wobei der Abtrennungsschritt die Isolation des PrP des an den Träger gebundenen Komplexes durch ein affinitätschromatographisches Elutionsverfahren umfasst.

20. Verfahren nach Anspruch 19, **dadurch gekennzeichnet, dass** die Isolation durch Elution des an den festen Träger fixierten PrP vermittels eines Puffers mit einem pH-Wert von 2 bis 10,5 und einer NaCl-Konzentration von 0 bis 5 M, vorzugsweise mit einem Glycin-HCl-Puffer von 0,1 Mol pro Liter mit einem pH-Wert von 2,5, durchgeführt wird.

21. Verfahren nach einem der Ansprüche 1 bis 6 zur Abtrennung und/oder Detektion und/oder Identifikation und/oder Quantifikation von mindestens einem PrP in einem biologischen Material, das von Natur aus mindestens eine Form von β2GPI enthält, **dadurch gekennzeichnet, dass** es einen Schritt der Abtrennung und/oder Detektion und/oder Identifikation und/oder Quantifikation eines PrP/β2GPl-Komplexes umfasst, gebildet aus mindestens einem an mindestens eine natürlich in diesem Material vorkommende β2GPI-Form gebundenen PrP.
